# EUROPEAN PATENT APPLICATION

(11) **EP 1 418 522 A2**
(43) Date of publication of application: **12.05.2004**
(21) Application number: 03025029.4
(22) Date of filing: 30.10.2003
(51) Int. Cl.: G06F 19/00

(54) **Medical information system and medical information management method**

(30) Priority: 05.11.2002 JP 2002321148
(71) Applicant: Olympus Corporation, Tokyo 151-0072 (JP)
(72) Inventor: Hirose, Koichi, Hino-shi Tokyo 191-0011 (JP)
(74) Representative: von Hellfeld, Axel, Dr. Dipl.-Phys.

(57) **Abstract**

A medical information system comprises an inputting unit inputting report information on an examination, a first storing unit storing the report information input by the inputting unit, and a creating unit creating explanatory information to a patient by using the report information.

## Description

### Cross Reference to Related Applications

This application is based upon and claims the benefit of priority from the prior Japanese Patent Application No. 2002-321148, filed Nov. 05, 2002, the entire contents of which are incorporated herein by reference.

### Background of the Invention

### Field of the Invention

The present invention relates to a medical information system and a medical information management method, which manage information of a report, etc. on an examination.

### Description of the Related Art

Conventionally, in a hospital, etc., a report on an examination is input by a responsible doctor via a PC (Personal Computer) terminal, etc. upon termination of the examination conducted for a patient, and information about that report is managed by being corresponded to the patient as electronic data. This information is a report for a doctor or a hospital, and for example, a pathology request form or a medical care information provision form, etc. are created based on this information.

In the meantime, there are cases where a request to provide an examination result (including an explanation) is made from a patient upon termination of the examination. In this case, for example, if the examination is an endoscopy, a shot endoscopic image is provided by being printed with a video printer at most, and its explanatory document is rarely added. Even if an explanatory document is provided, the above described report is provided unchanged as the explanatory document, or an examination result is provided in a way such that a doctor separately creates an explanatory document to give an explanation to a patient.

However, the above described report is created for a doctor or a hospital, and is not intended to give an explanation to a patient. Accordingly, its contents are represented by technical medical terms in many cases, and a patient cannot sufficiently understand the contents if he or she only views the report provided as the above described explanatory document.

Additionally, if a doctor separately creates an explanatory document to give an explanation to a patient as described above, this imposes a heavier load on the doctor, leading to a decrease in an operation efficiency.

### Summary of the Invention

A preferred embodiment according to the present invention is a medical information system comprising: an inputting unit inputting report information on an examination; a first storing unit storing the report information input by the inputting unit; and a creating unit creating explanatory information to a patient by using the report information.

### Brief Description of the Drawings

Fig. 1 exemplifies the configuration of a medical information system according to a preferred embodiment of the present invention;
Fig. 2 exemplifies the configuration of an endoscopy system;
Fig. 3 exemplifies the configuration of a report inputting/outputting terminal;
Fig. 4 exemplifies an input screen for creating a report, which is displayed on a PC monitor of the report inputting/outputting terminal, when a report information creation process is started;
Fig. 5 is a flowchart exemplifying an input process executed when an input is made to each of input fields such as "findings", "diagnosis", and "procedures" on the input screen;
Fig. 6 exemplifies a term selection window 50;
Fig. 7 exemplifies the term selection window 50;
Fig. 8 exemplifies the input screen;
Fig. 9 exemplifies the input screen displayed by executing the process shown in Fig. 5 when an observation tag of "larynx" is selected;
Fig. 10 exemplifies the input screen displayed by executing the process shown in Fig. 5 when an observation tag of "stomach" is selected;
Fig. 11 exemplifies the input screen displayed by executing the process shown in Fig. 5 when an observation tag of "duodenum" is selected;
Fig. 12 exemplifies an endoscopy report screen;
Fig. 13 exemplifies an explanatory screen to a patient;
Fig. 14A is a flowchart exemplifying a display process executed on the explanatory screen to a patient;
Fig. 14B is a flowchart exemplifying a display process executed on the explanatory screen to a patient;
Fig. 15A is a flowchart exemplifying a display process executed on the explanatory screen to a patient;
Fig. 15B is a flowchart exemplifying a display process executed on the explanatory screen to a patient;
Fig. 16A exemplifies a pop-up menu;
Fig. 16B exemplifies a word explanation window;
Fig. 17A exemplifies a pop-up menu;
Fig. 17B exemplifies a pop-up menu;
Fig. 18 exemplifies an accompanying information screen; and
Fig. 19 exemplifies a printing result based on explanatory information to a patient.

### Description of the Preferred Embodiment

A preferred embodiment according to the present invention is described below with reference to the drawings.

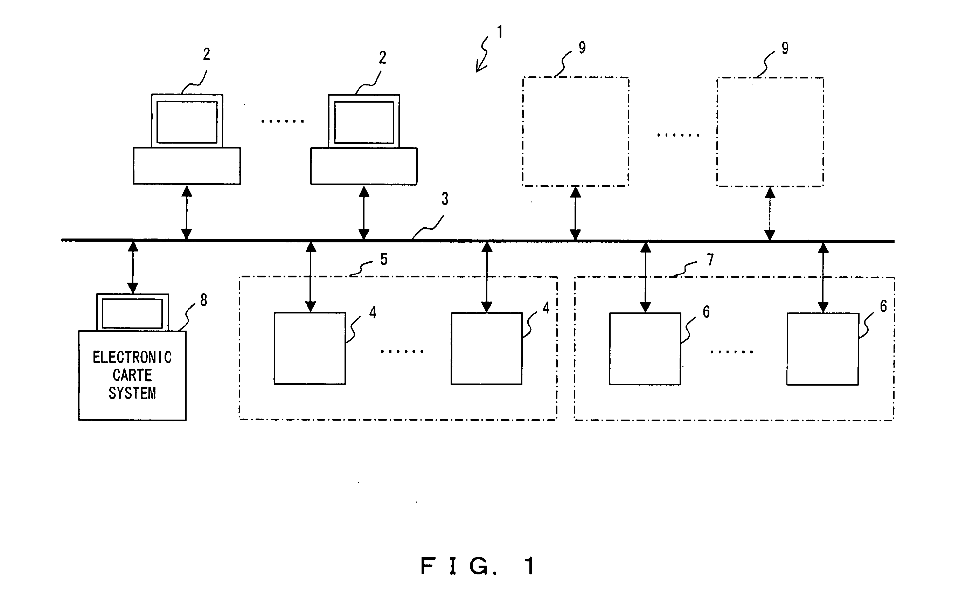

Fig. 1 exemplifies the configuration of a medical information system according to the preferred embodiment of the present invention.

As shown in this figure, the medical information system 1 according to the preferred embodiment of the present invention comprises: a plurality of in-hospital information terminals 2 provided in respective outpatient departments within a hospital; an endoscopic department 5 composed of a plurality of endoscopy systems 4; a pathologic department 7 composed of a plurality of pathologic examination systems 6; an electronic carte system 8 storing patient data input from the in-hospital information terminals 2, examination data from the endoscopic department 5 and the pathologic department 7, etc. in a predetermined format; and the like. These constituent elements are configured to be able to exchange data mutually by being connected to a hospital-wide network 3.

Additionally, to the hospital-wide network 3, examination systems 9 of other departments, such as an ultrasonic examination system, a CT system, an MRI system, and the like can be connected, although they are not shown.

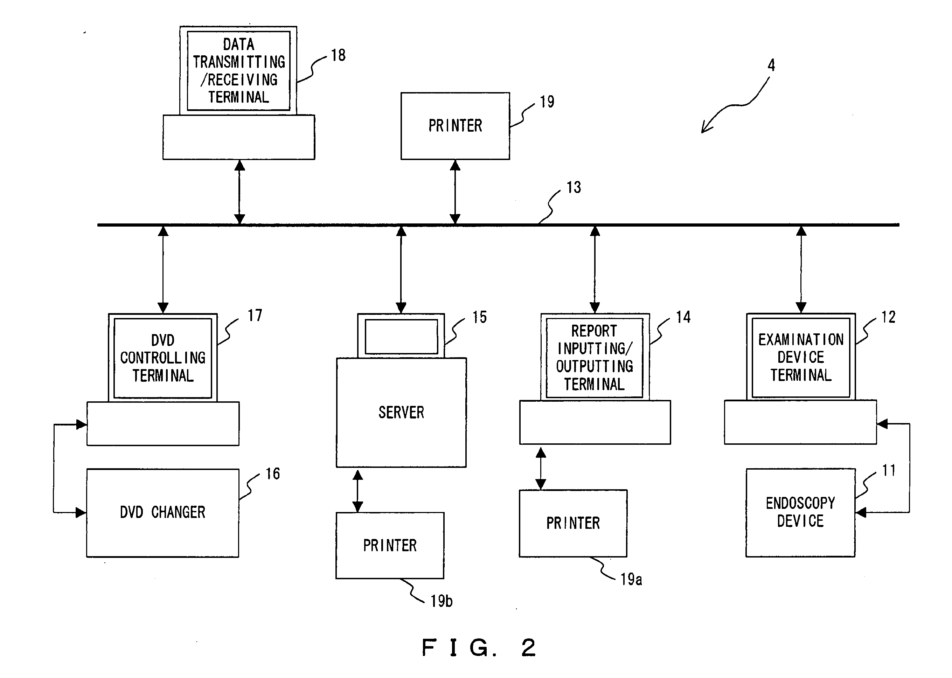

Fig. 2 exemplifies the configuration of the above described endoscopy system 4.

As shown in this figure, the endoscopy system 4 comprises: an examination device terminal 12 connected to an endoscopy device 11 conducting an endoscopy within a body cavity; a report inputting/outputting terminal 14 inputting report information such as an endoscopy result, etc., and creating explanatory information to a patient based on the report information; a server 15 storing examination data (endoscopic image data, etc.) from the examination device terminal 12, the report information and the explanatory information to a patient based on the report information from the report inputting/outputting terminal 14, and the like; a DVD controlling terminal 17 controlling a DVD changer 16, which is a large-capacity storage device for backing up the information stored in the server 15; a data transmitting/receiving terminal 18 monitoring the electronic carte system 8, and managing the transmission/reception of examination request data, examination result data, etc.; a printer 19 printing an examination report based on report information, contents based on the explanatory information to a patient, etc.; and the like. These constituent elements are configured to be able to exchange data mutually by being connected to a system network 13. Additionally, printers 19a and 19b are respectively connected to the server 15 and the report inputting/outputting terminal 14, and predetermined printing can be made depending on need.

Also the pathologic examination system 6 and the examination systems 9 of the other departments have a configuration similar to that of such an endoscopy system 4, although this is not shown.

The respective terminals such as the above described in-hospital information terminals 2, examination device terminal 12, report inputting/outputting terminal 14, DVD controlling terminal 17, etc. are configured by a personal computer (PC).

Here, configuration of the report inputting/outputting terminal 14 is described as one example of the configuration.

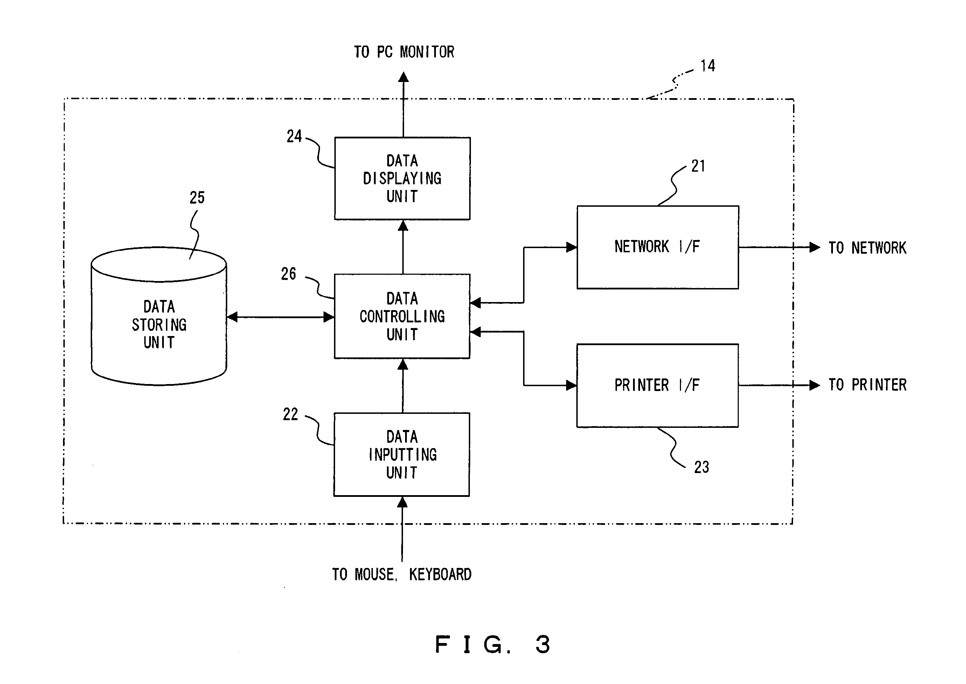

Fig. 3 exemplifies the configuration of the report inputting/outputting terminal 14.

As shown in this figure, the report inputting/outputting terminal 14 is configured by comprising: a network I/F 21, which is connected to the system network 13, transmitting/receiving data; a data inputting unit 22 connected to a data input device such as a mouse, a keyboard, etc.; a printer I/F 23 connected to a data output device such as a printer, etc.; a data displaying unit 24 connected to a PC monitor; a data controlling unit 26 controlling the network I/F 21, the data inputting unit 22, the printer I/F 23, and the data displaying unit 24, executing a data process, etc., and storing a process result in a data storing unit 25 such as a hard disk, etc.; and the like.

A medical information management process executed in the medical information system 1 having such a configuration is described below.

For example, when endoscopy request information is issued by an in-hospital information terminal 2, it is received by the data transmitting/receiving terminal 18 of an endoscopy system 4. The received endoscopy request information is registered to a database within the server 15 by the data transmitting/receiving terminal 18.

Then, in the endoscopy system 4, an examination by the endoscopy device 11 is conducted according to the endoscopy request information registered to the server 15. Specifically, the examination device terminal 12 connected to the endoscopy device 11 obtains the endoscopy request information from the server 15, so that the endoscopy can be started.

When the examination is terminated, for example, by shooting an endoscopic image with the endoscopy device 11, the endoscopic image is stored in the server 15 by the examination device terminal 12 by being associated with the endoscopy request information. Besides, when the endoscopic image is stored (registered) in the server 15, it is copied to the DVD changer 16 by the DVD controlling terminal 17.

Additionally, when report information on the conducted examination is input to the report inputting/outputting terminal 14, explanatory information to a patient is created based on the report information by the report inputting/outputting terminal 14. The report information, and the explanatory information to a patient, which is created based on the report information, are registered to the database within the server 15 by being associated with the endoscopy request information. The registered report information is never erased from the database within the server 15 thereafter. Besides, when the report information is registered (recorded) to the server 15, it is copied to the DVD changer 16 by the DVD controlling terminal 17.

Furthermore, the report information is also transmitted to the electronic carte system 8 the same time the report information, and the explanatory information to a patient, which is created based on the report information, are registered to the server 15 by the report inputting/outputting terminal 14.

Still further, the report information registered to the database of the server 15 in this way is called by the report inputting/outputting terminal 14 depending on need, and allowed to be referenced or edited.

A report information creation process executed by the report inputting/outputting terminal 14 is described next. This report information creation process is a process executed when report information on a conducted examination is input. This process is executed in a way such that the data controlling unit 26 reads out and executes a control program stored in an internal memory.

Once the report information creation process is started, an input screen for creating a report is first displayed on a PC monitor of the report inputting/outputting terminal 14. On this screen, a desired input, instruction, etc. is made, whereby report information on an examination can be input.

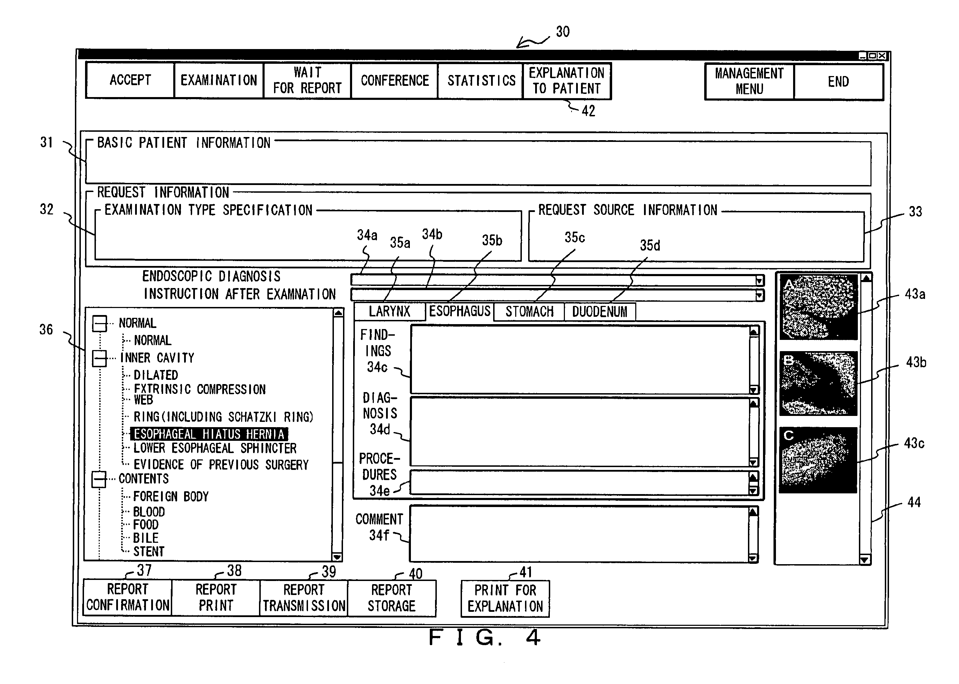

Fig. 4 exemplifies the input screen for creating a report, which is displayed on the PC monitor of the report inputting/outputting terminal 14 when the report information creation process is started.

The input screen shown in this figure depicts the input screen 30 for creating a report on an upper gastrointestinal endoscopy. This input screen 30 is configured mainly by: a display area 31 in which basic patient information is displayed; a display area 32 in which examination type specification is displayed; a display area 33 in which request source information is displayed; report information input fields 34 (34a, 34b, 34c, 34d, 34e, and 34f) to which examination result information (report information) is input; observation tags 35 (35a, 35b, 35c, and 35d), with which an organ to be observed can be specified; a term selection field 36 in which a term can be selected; a report confirmation button 37 for making an instruction to confirm a created report; a report print button 38 for making an instruction to print a created report; a report transmission button 39 for making an instruction to transmit/store created report information and explanatory information to a patient based on the report information to/in the server 15, and to transmit/store the report information to/in the electronic carte system 8; a report storage button 40 for making an instruction to temporarily store created report information and explanatory information to a patient based on the report information in the server 15; a print-for-explanation button 41 for making an instruction to print contents based on explanatory information to a patient; an explanation-to-patient button 42 for making an instruction to display an explanatory screen to a patient; an endoscopic image field 44 in which endoscopic images 43 (43a, 43b, and 43c in this figure) shot in an endoscopy are displayed; and the like.

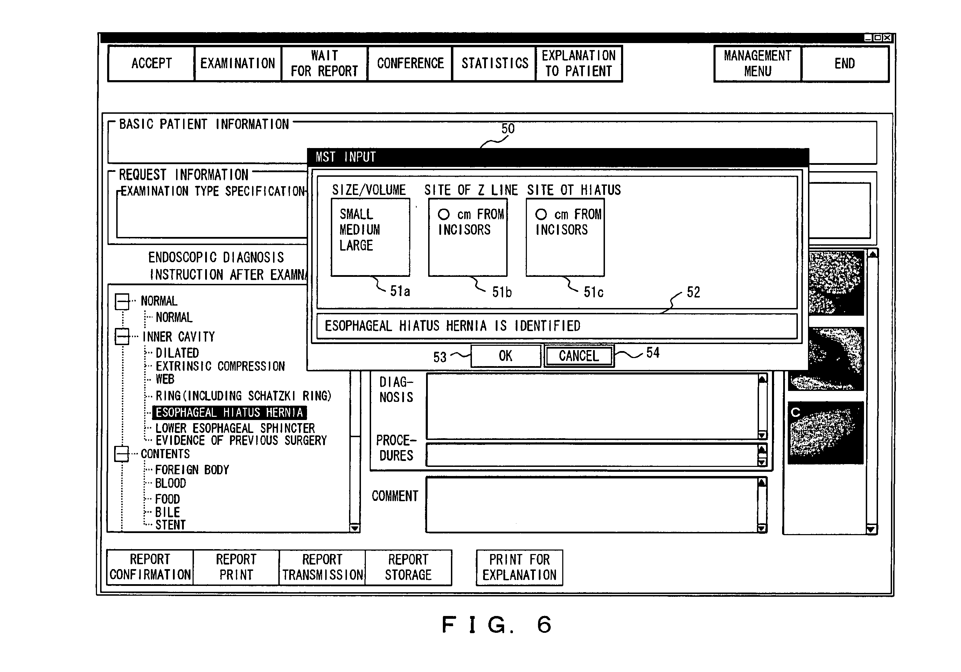

Contents displayed in the respective display areas 31 to 33 (not shown in this figure) are displayed based on information obtained from the database of the server 15. Additionally, the report information input fields 34 are composed of the input fields 34a to 34f such as "endoscopic diagnosis", "instruction after examination", "findings", "diagnosis", "procedures", and "comment", and inputs can be respectively made to the input fields 34a to 34f. Furthermore, the observation tags 35 are composed of the observation tags 35a to 35d such as "larynx", "esophagus", "stomach", and "duodenum". Any of the observation tags 35 is selected, which enables an input to the input fields 34c to 34e for the corresponding organ to be observed. The example shown in this figure depicts the state where the observation tag 35b is selected, and an input to each of the input fields 34c to 34e such as "findings", "diagnosis", and "procedures" for "esophagus" can be made. Besides, a text where terms selected in a term selection window 50 (to be described later with reference to Fig. 6), which is displayed by selecting a desired term from a term list displayed in the term selection field 36, are combined is input to each of the input fields 34c to 34e. Terms displayed in the term selection field 36 are terms that are preregistered (prerecorded) to the database of the server 15, and conform, for example, to MST (Minimal Standard Terminology). Corresponding terms are read out and displayed in the term selection field 36 depending on need.

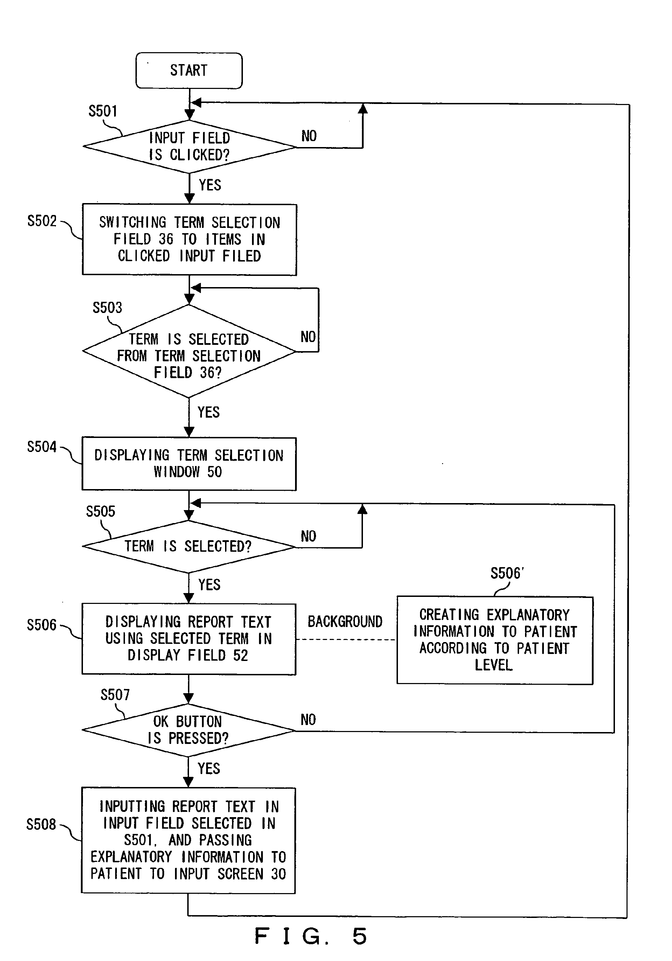

Fig. 5 is a flowchart exemplifying an input process executed when an input is made to the input fields 34c to 34e on such an input screen 30.

In this process, an input is made to an organ to be observed, which corresponds to an observation tag 35 being selected. When the input screen 30 is activated, the observation tag 35a ("larynx") is selected by default.

This example assumes that the observation tag 35b ("esophagus") is selected as shown in Fig. 4.

In the input process shown in Fig. 5, firstly, in S501, it is determined whether or not any of the input fields 34c to 34e is clicked with a mouse. If the determination results in "YES", the process goes to S502. If the determination results in "NO", this step is repeated.

In S502, a term list that corresponds to the input field clicked in the preceding step is displayed in the term selection field 36. Namely, contents displayed in the term selection field 36 are switched to the term list that corresponds to the input field clicked. In the example shown in Fig. 4, the input field 34c ("findings") is clicked, and the term list that corresponds to this field is displayed in the term selection field 36.

In S503, it is determined whether or not a desired term is selected from the term list displayed in the term selection field 36. If the determination results in "YES", the process goes to S504. If the determination results in "NO", this step is repeated.

In S504, the term selection window 50, which corresponds to the term selected in the preceding step, is displayed.

Fig. 6 exemplifies the term selection window 50 displayed at this time. The term selection window 50 shown in this figure is displayed when the input field 34c ("findings") is clicked, and "esophageal hiatus hernia" is selected from the term list displayed in the term selection field 36 in correspondence with the input field 34c. The term selection window 50 is configured by: term selection item fields 51 composed of an input field 51a in which selectable terms are displayed, and input fields 51b and 51c, to which numerical values of a length, etc. are input; a text display field 52 where a default report text, or a report text, which is created based on a selected term, input numerical values, etc. in the respective term selection item fields 51, is displayed; an OK button 53; a cancel button; and the like.

Selectable terms displayed in the term selection item field 51 are terms pre-registered (prerecorded) to the database of the server 15. Corresponding terms are read out and displayed in the term selection item field 51 depending on need. Besides, a report text displayed in the text display field 52 is created based on a selected term and input numerical values in the above described term selection item fields 51, and terms preregistered (prerecorded) to the database of the server 15.

In such a term selection window 52, a desired term is selected, and desired numerical values are input, whereby an input of "findings" for "esophageal hiatus hernia" is made.

Also the term selection window 50, which corresponds to each of the terms displayed in the term selection field 36, is configured by corresponding term selection item fields 51, text display area 52, OK button 53, cancel button 54, etc., and an input can be made in a similar manner.

Then, in S505, it is determined whether or not a term is selected, and numerical values are input in the respective term selection item fields 51 of the term selection window 50. If the determination results in "YES", the process goes to S506. If the determination results in "NO", this step is repeated.

In S506, a report text is created based on the term and the numerical values, which are selected and input in the respective term selection item fields 51, and displayed in the text display field 52.

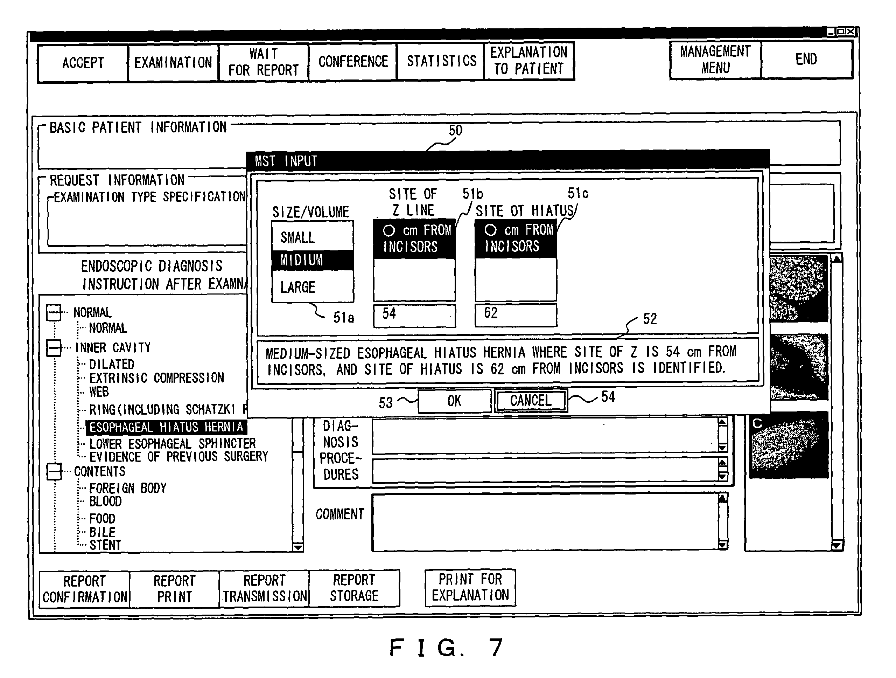

Fig. 7 exemplifies the term selection window 50 displayed at this time. The example shown in this figure is an example where a term "medium" selected in the term selection item field 51a is highlighted, numerical values "54" and "62" input in the term selection item fields 51b and 51c are displayed, and a report text that is created based on the term and the numerical values is displayed in the text display field 52.

Additionally, in S506' executed in the background of step S506, explanatory information to a patient according to the medical knowledge level of a patient is also created from the created report text based on information about the medical knowledge level (the degree of understanding) of the patient, which is recorded in the server 15. However, if the patient is a newcomer, the information about the medical knowledge level of the patient is unregistered. Therefore, explanatory information to a patient according to a standard (medium) level is created in this case.

In S507, it is determined whether or not the OK button 53 is clicked. If the determination results in "YES", the process goes to S508. If the determination results in "NO", the process goes back to S505.

In S508, the term selection window 50 is closed, and the report text created in the above described S506 is input to the input field 34 clicked in the above descried S501. Besides, the explanatory information to a patient, which is created in S506', is passed to the input screen 30.

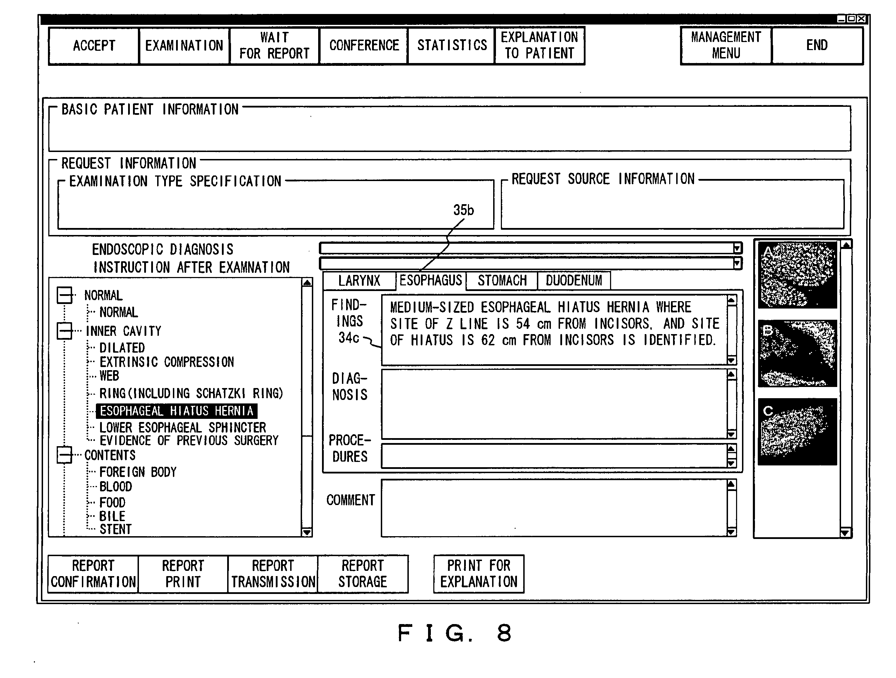

Fig. 8 exemplifies the input screen 30 displayed at this time. The example shown in this figure is an example where the report text created in the term selection window 50 shown in Fig. 7 is input to the report information input field 34c of the input screen 30.

Upon termination of the process of S508, the process goes back to S501.

The processes in S501 to S508 are repeatedly executed in this way, whereby an input of a corresponding report text can be made to each of the input fields 34c to 34e such as "findings", "diagnosis", and "procedures" for "esophagus".

Additionally, the process shown in Fig. 5 is executed when each of the other observation tags 35a, 35c, and 35d is selected, whereby an input of a report text to each of "findings", "diagnosis", and "procedures" for the other organs to be observed, such as "larynx", "stomach", and "duodenum" can be made.

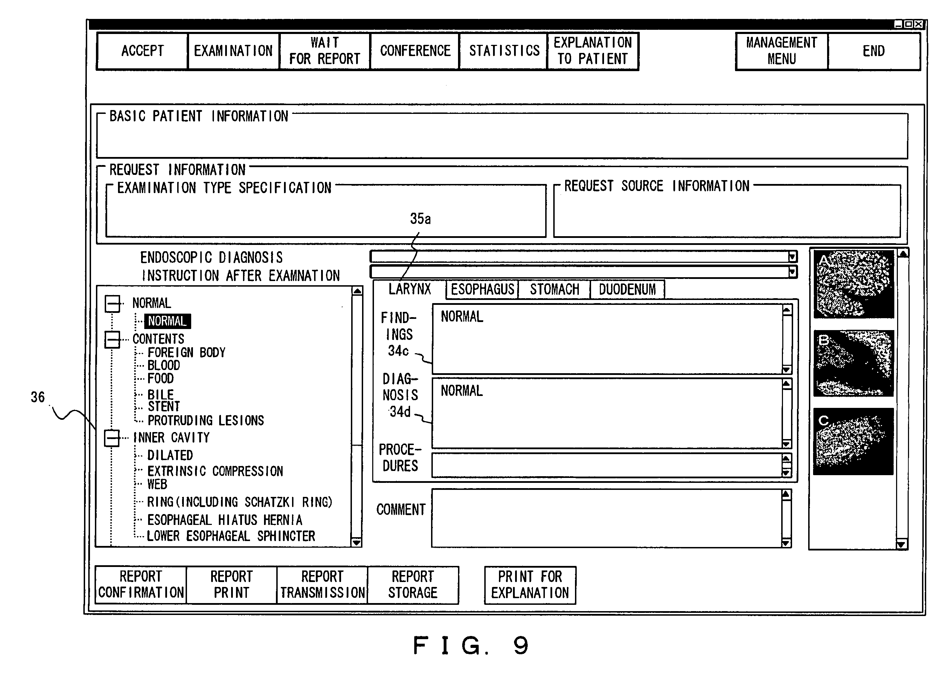

Fig. 9 exemplifies the input screen 30 displayed by executing the process shown in Fig. 5 when the observation tag 35a "larynx" is selected. The example shown in Fig. 9 depicts an example where a report text is input to the input fields 34c and 34d such as "findings" and "diagnosis" for "larynx". Additionally, a corresponding term list is displayed in the term selection field 36 as a result of clicking the input field 34c ("findings").

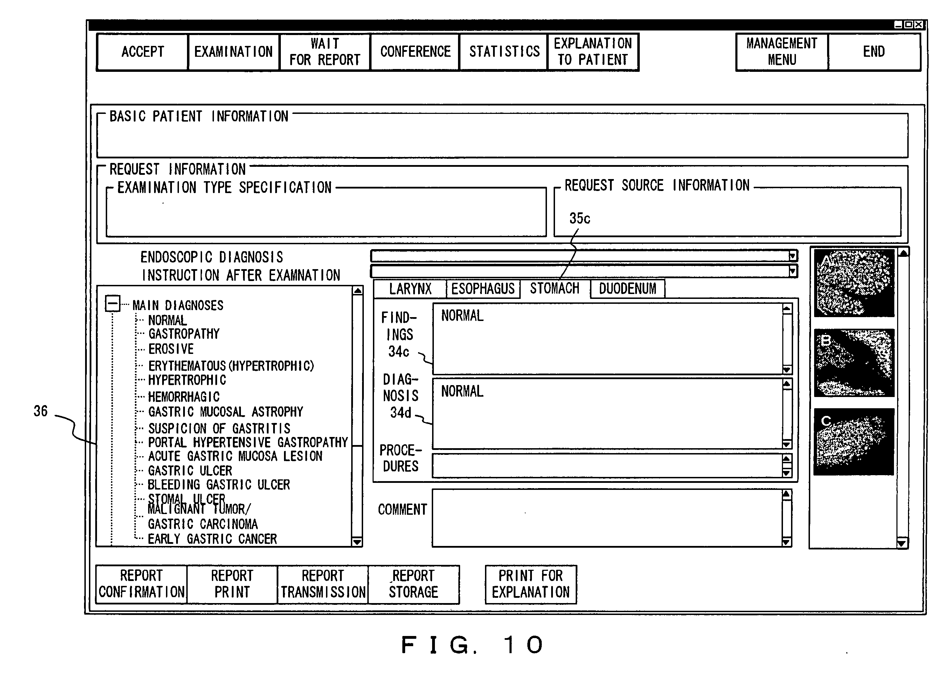

Fig. 10 exemplifies the input screen 30 displayed by executing the process shown in Fig. 5 when the observation tag 35c "stomach" is selected. The example shown in this figure depicts an example where a report text is input to the input fields 34c and 34d such as "findings" and "diagnosis" for "stomach". Besides, a corresponding term list is displayed in the term selection field 36 as a result of clicking the input field 34d ("diagnosis").

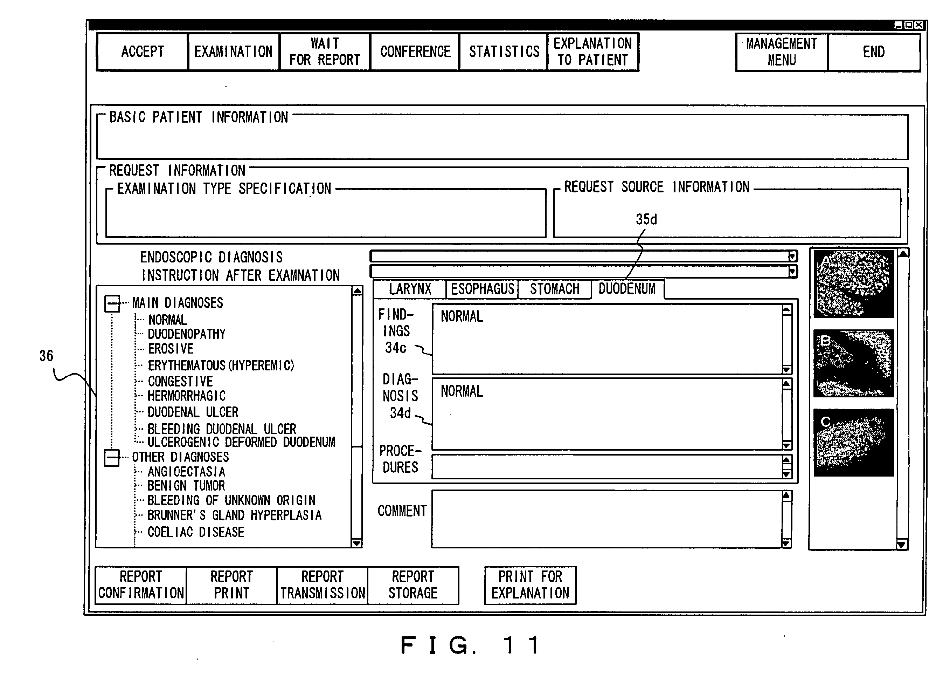

Fig. 11 exemplifies the input screen 30 displayed by executing the process shown in Fig. 5 when the observation tag 35d "duodenum" is selected. The example shown in Fig. 11 depicts an example where a report text is input to the input fields 34c and 34d such as "findings" and "diagnosis" for "duodenum". Besides, a corresponding term list is displayed in the term selection field 36 as a result of clicking the input field 34d ("diagnosis").

For the input screens 30 shown in Figs. 9 to 11, the examples where no input is made to the input field 34e "procedures" are shown. Naturally, however, an input can be also made to this input field 34e in a similar manner by executing the process shown in Fig. 5

Additionally, on the input screen 30, a text input can be directly made to the report information input fields 34a ("endoscopic diagnosis''), 34b ("instruction after examination''), and 34f ("comment") with the click of each of the input fields 34. Besides, to the input fields 34a and 34b, an input can be also made by selecting a sample text from a pull-down menu that is displayed with the press (click) of a button at the right of each of the fields. One or more of the sample text can be pre-registered in the system and displayed in the pull-down menu(s). As is known in the art, selecting a sample text from the pull-down menu automatically inserts the same into the corresponding input field 34a, 34b.

As described above, each of the observation tags 35 is selected, a report text is input to each of the input fields 34c to 34e such as "findings", "diagnosis", and "procedures" for each of the organs to be observed, such as "larynx", "esophagus", "stomach", and "duodenum", a desired input is made to each of the report information input fields 34a, 34b, and 34f, and the report confirmation button 37 is pressed (clicked), so that an endoscopy report screen 60 indicating the report that is created based on the information, etc. input to the input fields 34a to 34f so far is displayed on the input screen 30. With this screen, the created report can be confirmed.

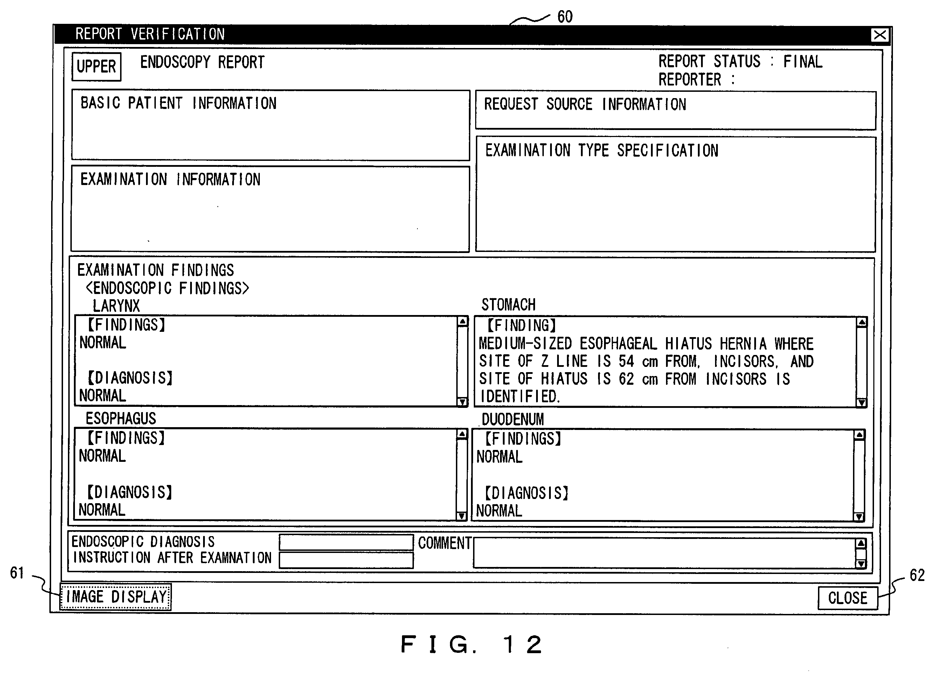

Fig. 12 exemplifies the endoscopy report screen 60 displayed at this time. As shown in this figure, report texts, which are input to the respective input fields 34c to 34e such as "findings", "diagnosis", and "procedures" for each of the organs to be observed such as "larynx", "esophagus", "stomach", and "duodenum", and to the input fields 34a, 34b, and 34f such as "endoscopic diagnosis", "instruction after examination, and "comment" on the input screen 30, are displayed as a list on the endoscopy report screen 60, with which the created report can be confirmed at a glance.

Additionally, the endoscopic images 43 shown in Fig. 4, etc. are displayed with the press (click) of an image display button 61 on the endoscopy report screen 60. The endoscopy report screen 60 is closed with the press (click) of a close button 62.

Furthermore, with the press (click) of the report print button 38 on the input screen 30, the created report is printed. Besides, with the press (click) of the report storage button 40, the created report information, and explanatory information to a patient, which is created based on the report information, are temporarily stored in the server 15. Or, with the press (click) of the report transmission button 39, the created report information, and the explanatory information to a patient, which is created based on the report information, are stored (registered) in the server 15, and at the same time, the report information is transmitted to the electronic carte system 8.

Still further, a predetermined instruction is issued, whereby report information stored in the server 15 can be also edited, although this is not shown. Here, if report information is edited, explanatory information to a patient is created based on the edited report information. However, in this case, the report information before being edited, and explanatory information to a patient based on the report information before being edited are newly created without being overwritten (updated) by the report information after being edited, and the explanatory information to a patient based on the report information after being edited, and processes in (1) and (2) to be described next are executed each time. As a result, the version number of the report information is upgraded.

Firstly, in (1), by the report inputting/outputting terminal 14, report information after being edited, and explanatory information to a patient, which is created based on the report information after being edited, are registered (stored) in the database within the server 15 by being associated with endoscopy request information, and at the same time, the report information after being edited is transmitted to the electronic carte system 8.

In (2), the report information after being edited, and the explanatory information to a patient created based on the report information, both of which are registered to the server 15, are copied to the DVD changer 16 by the DVD controlling terminal 17.

Additionally, the report information, which is transmitted from the report inputting/outputting terminal 14 to the electronic carte system 8, is registered to a database of the electronic carte system 8 by the electronic carte system 8. The report information registered to the database of the electronic carte system 8 in this way can be displayed and referenced by an in-hospital information terminal 2. However, the report information cannot be edited by an in-hospital information terminal 2.

Additionally, with the press (click) of the explanation-to-patient button 42 on the input screen 30, an explanatory screen to a patient 70 is displayed. Note that the explanation-to-patient button 42 is pressed, for example, when a doctor explains an examination result to a patient.

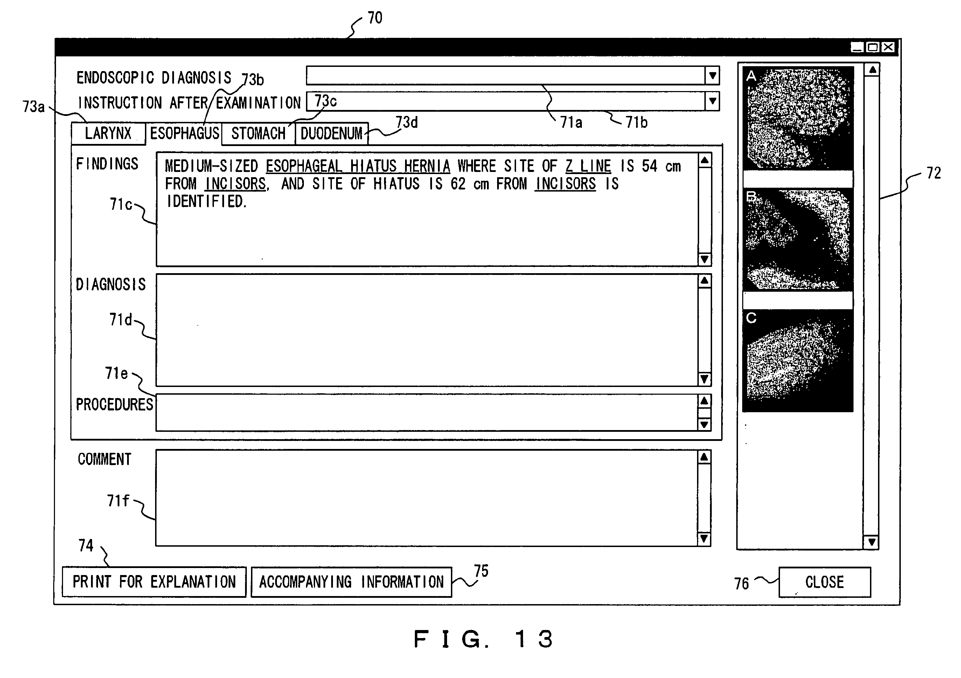

Fig. 13 exemplifies the explanatory screen to a patient 70 displayed at this time. On the explanatory screen to a patient 70 shown in this figure, contents displayed in or corresponding to each of the report information input fields 34a to 34f, and the endoscopic image field 44 on the input screen 30 are displayed in each of an endoscopic diagnosis field 71a, an instruction-after-examination field 71b, a findings field 71c, a diagnosis field 71d, a procedures field 71e, a comment field 71f, and an image field 72. More specifically, contents based on the explanatory information to a patient (explanatory information to a patient, which is created with the above described process in S506' of Fig. 5) according to information about the medical knowledge level of a patient, which is created from a report text displayed in each of the report information input fields 34c to 34e, are displayed in each of the findings field 71c, the diagnosis field 71d, and the procedures field 71e. For the fields other than these fields, the contents displayed on the input screen 30 are displayed.

The example shown in Fig. 13 is a display example where a medical knowledge level is a standard (medium) level in the information about the medical knowledge level of a patient, and the contents based on the explanatory information to a patient, which are displayed, for example, in the findings field 71c on the explanatory screen to a patient 70, are the same as those of the report information input to the report information input field 34c for the corresponding organ (esophagus) on the input screen 30.

Additionally, on the explanatory screen to a patient 70, observation tags 73 (73a, 73b, 73c, and 73d), with which an organ to be observed can be specified, are provided similar to the input screen 30. In the example shown in Fig. 13, the observation tag 73b ("esophagus") is selected (clicked), and contents of "findings", "diagnosis" and "procedures" for "esophagus" are displayed. When the explanatory screen to a patient 70 is activated, the observation tag 73a ("larynx'') is selected by default.

Furthermore, on the explanatory screen to a patient 70, a print-for-explanation button 74 is intended to make an instruction to print explanatory information to a patient, whereas an accompanying information button 75 is intended to make an instruction to display an accompanying information screen.

As described above, on the explanatory screen to a patient 70, contents based on explanatory information to a patient according to information about the medical knowledge level of a patient are first displayed in the findings field 71c, the diagnosis field 71d, and the procedures field 71e. However, these contents can be changed to a simpler or more technical expression, and displayed by making a predetermined instruction. More specifically, underlined words (such as "Z line", "incisors", "esophageal hiatus hernia", etc., which are displayed in the findings field 71c) within the contents based on the explanatory information to a patient displayed in the findings field 71c, the diagnosis field 71d, and the procedures field 71e, can be changed to words of simpler or more technical expressions, and displayed. Note that information about these words are respectively preregistered to the database of the server 15 by being corresponded to a document level, and read out depending on need, and corresponding words are displayed. This example is described by taking as an example the case where targets to be changed are words. However, a target to be changed may be a character, a text, etc.

Here, a display process executed on the explanatory screen to a patient 70 is described.

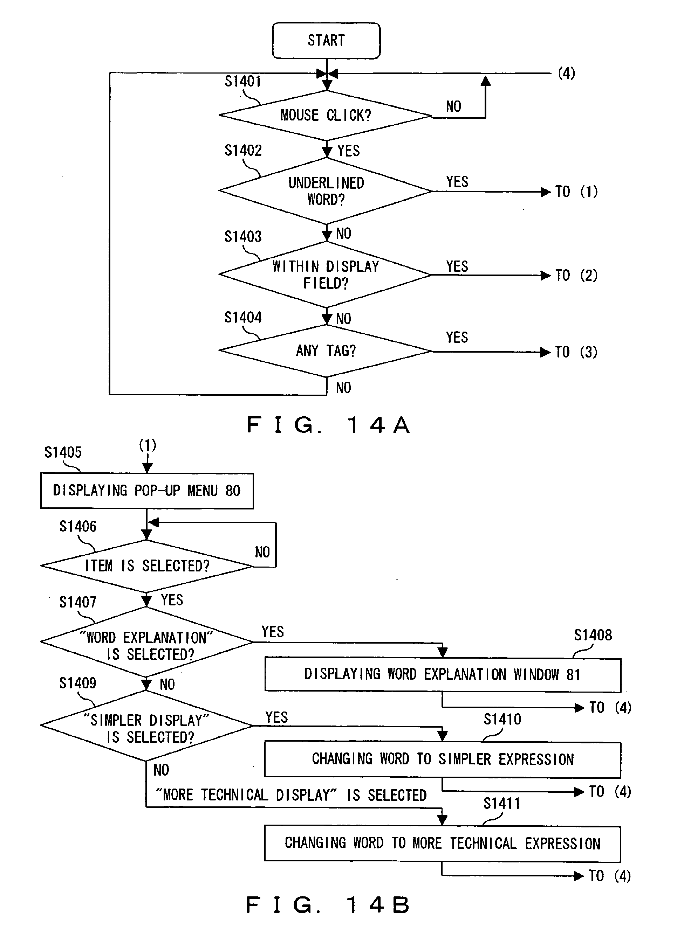

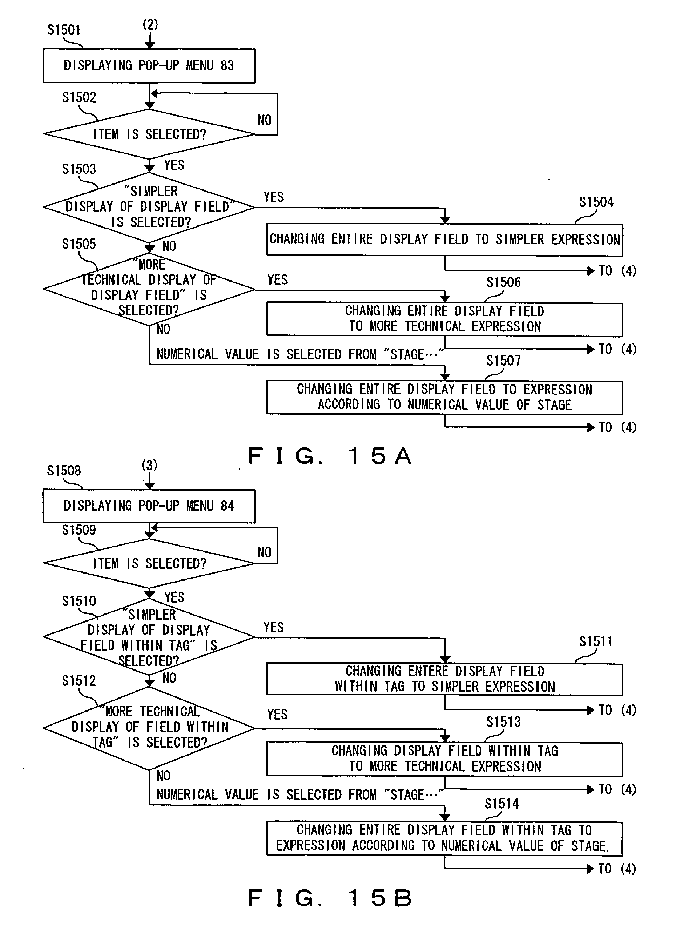

Figs. 14A, 14B, 15A, and 15B are flowcharts exemplifying the display process.

This example assumes that the observation tag 73b ("esophagus'') is selected when this display process is started.

Firstly, in S1401 of Fig. 14A, it is determined whether or not any on the explanatory screen to a patient 70 is clicked. If the determination results in "YES", the process goes to S1402. If the determination results in "NO", this step is repeated.

In S1402, it is determined whether or not the target clicked in the preceding step is an underlined word (such as "Z line", "incisors", or "esophageal hiatus hernia" displayed in the findings field 71c of Fig. 13) within the contents based on the explanatory information to a patient, which are displayed in the findings field 71c, the diagnosis field 71c, or the procedures field 71e. If the determination results in "YES", the process transfers to the process of (1) in Fig. 14B. If the determination results in "NO", the process goes to S1403.

In S1403, it is determined whether or not the target clicked in S1401 is any of the findings field 71c, the diagnosis field 71d, and the procedures field 71e. If the determination results in "YES", the process transfers to the process of (2) in Fig. 15A. If the determination results in "NO", the process goes to S1404.

In S1404, it is determined whether or not the target clicked in S1401 is any of the observation tags 73 (73a, 73b, 73c, and 73d). If the determination results in "YES", the process transfers to the process of (3) in Fig. 15B. If the determination results in "NO", the process goes back to S1401.

In S1404, executed may be a process for determining whether or not the target clicked in S1401 is the observation tag 73 (the observation tag 73b ("esophagus") in the example shown in Fig. 13) being selected, for transferring to the process of (3) in Fig. 15B if the determination results in "YES", or for going back to S1401 if the determination results in "NO".

With such a process shown in Fig. 14A, the display process for the clicked target is executed.

The above described process of (1) shown in Fig. 14B is described next.

In this figure, firstly, in S1405, a pop-up menu 80 for the word clicked in the above described S1401 is displayed.

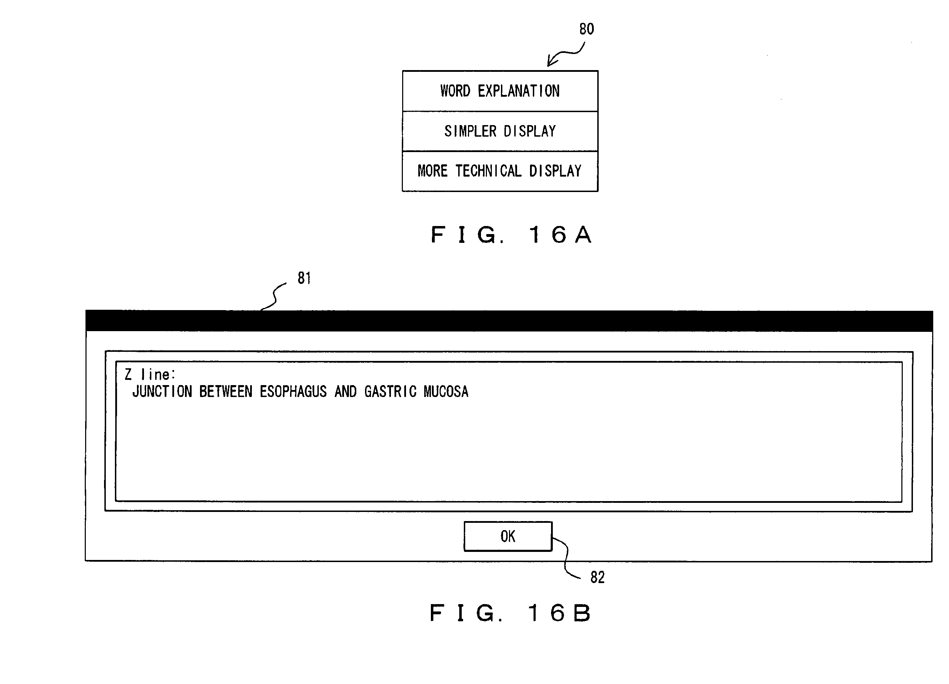

Fig. 16A exemplifies the pop-up menu 80 displayed at this time. As shown in this figure, in the pop-up menu 80, "word explanation", "simpler display", and "more technical display" are displayed as selectable items.

In S1406, it is determined whether or not any of the items "word explanation", "simpler display", and "more technical display" is selected. If the determination results in "YES", the process goes to S1407. If the determination results in "NO", this step is repeated.

In S1407, it is determined whether or not the item selected in the preceding step is the "word explanation". If the determination results in "YES", the process goes to S1408. If the determination results in "NO", the process goes to S1409.

In S1408, a word explanation window 81 is newly displayed, and the process goes back to (4), namely, S1401.

Fig. 16B exemplifies the word explanation window 81 displayed at this time. The example shown in this figure is a word explanation window 81 for the underlined word "Z line" within the text displayed in the findings field 71c on the explanatory screen to a patient 70 shown in Fig. 13. With this window, it can be verified that "Z line" is a "junction between the esophagus and the gastric mucosa". Such a word, and information about a corresponding word explanation are preregistered to the database of the server 15, read out according to a clicked word, and a word explanation is displayed. Additionally, this word explanation window 81 is closed with the press (click) of an OK button 82 provided in this window.

In S1409, it is determined whether or not the item selected in S1406 is the "simpler display". If the determination results in "YES", the process goes to S1410. If the determination results in "NO", the process goes to S1411.

In S1410, the word clicked in the above described S1401 is changed to a simpler expression, and displayed. Namely, the clicked word is changed to a word of a document level, which becomes an expression of the next simpler level of the document level corresponded to the word, and displayed. Then, the process goes back to (4), namely, S1401.

In S1411, it is proved that the item selected in S1406 is the "more technical display". Therefore, the word clicked in the above described S1401 is changed to a more technical expression, and displayed. Namely, the clicked word is changed to a word of a document level, which becomes an expression of the next more technical level of the document level corresponded to the word, and displayed. Then, the process goes back to (4), namely, S1401.

With such a process shown in Fig. 14B, according to an item selected from the pop-up menu 80, an explanation of a word clicked in S1401 is displayed, or the word is changed to a word of a simpler or more technical expression, and displayed.

The above described process of (2) shown in Fig. 15A is described next.

In this figure, firstly, in S1501, a pop-up menu 83 for any of the findings field 71c, the diagnosis field 71d, and the procedures field 71e, which is clicked in the above described S1401, is displayed.

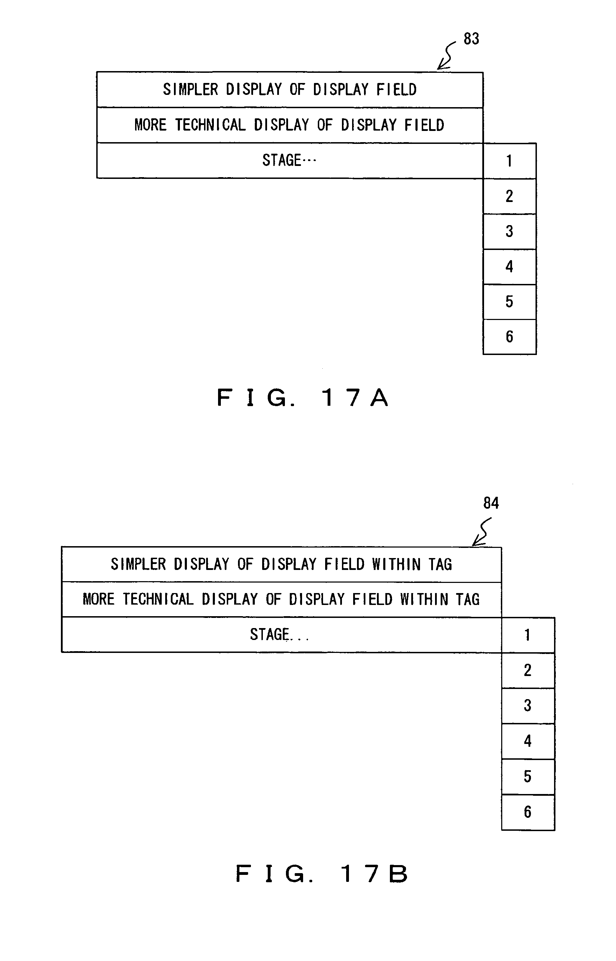

Fig. 17A exemplifies the pop-up menu 83 displayed at this time. As shown in this figure, in the pop-up menu 83, "simpler display of display field", "more technical display of display field", and "stage..." are displayed as selectable items. Additionally, for the "stage...", [1], [2], [3], [4], [5], and [6], which indicate a document level, are further displayed as selectable items. In this example, the document levels become more technical as they ascend from [1] to [6] (or the document levels become simpler as they descend from [6] to [1]).

In S1502, it is determined whether or not any of the items "simpler display of display field", "more technical display of display field", and [1], [2], [3], [4], [5], and [6] of "stage..." is selected. If the determination results in "YES", the process goes to S1503. If the determination results in "NO", this step is repeated.

In S1503, it is determined whether or not the item selected in the preceding step is "simpler display of display field". If the determination results in "YES", the process goes to S1504. If the determination results in "NO", the process goes to S1505.

In S1504, contents based on the explanatory information to a patient, which are displayed in any of the findings field 71c, the diagnosis field 71d, and the procedures field 71e, which is clicked in the above described S1401, are changed to a simpler expression, and displayed. Namely, each underlined word within the contents is changed to a word of a document level, which becomes an expression of the next simpler level of the document level corresponding to the word, and displayed. Then, the process goes back to (4), namely, S1401.

In S1505, it is determined whether or not the item selected in S1502 is "more technical display of display field". If the determination results in "YES", the process goes to S1506. If the determination results in "NO", the process goes to S1507.

In S1506, contents based on the explanatory information to a patient, which are displayed in any of the findings field 71c, the diagnosis field 71d, and the procedures field 71e, which is clicked in the above described S1401, is changed to a more technical expression, and displayed. Namely, each underlined word within the contents is changed to a word of a document level, which becomes an expression of the next more technical level of the document level corresponding to the word, and displayed. Then, the process goes back to (4), namely, S1401.

In S1507, it is proved that the item selected in S1502 is any of the document levels [1], [2], [3], [4], [5], and [6] of the "stage...". Therefore, contents based on the explanatory information to a patient, which are displayed in any of the findings field 71c, the diagnosis field 71d, and the procedures field 71e, are changed to an expression according to the document level selected in the "stage..." and displayed. Namely, each underlined word within the contents is changed to a word according to the document level selected in the "stage...", and displayed. Then, the process goes back to (4), namely, S1401.

With such a process shown in Fig. 15A, contents of any of the findings field 71c, the diagnosis field 71d, and the procedures field 71e, which is clicked in S1401, are changed to contents of a simpler or more technical expression, or to contents of an expression according to a desired document level, and displayed.

The above described process of (3) shown in Fig. 15B is described next.

In this figure, firstly, in S1508, a pop-up menu 84 for the observation tag 73 (73a, 73b, 73c, or 73d) clicked in the above described S1401 is displayed.

Fig. 17B exemplifies the pop-up menu 84 displayed at this time. As shown in this figure, in the pop-up menu 84, "simpler display of display field within a tag", "more technical display of display field within a tag", and "stage..." are displayed as selectable items. Additionally, in the "stage...", [1], [2], [3], [4], [5], and [6], which indicate a document level, are further displayed as selectable items. In this example, the document levels become more technical as they ascend from [1] to [6] (or the document levels become simpler as they descend from [6] to [1]).

In S1509, it is determined whether or not any of the items "simpler display of display field within a tag", "more technical display of display field within a tag", and [1], [2], [3], [4], [5], and [6] of the "stage..." is selected from the pop-up menu 84. If the determination results in "YES", the process goes to S1510. If the determination results in "NO", this step is repeated.

In S1510, it is determined whether or not the item selected in the preceding step is "simpler display of display field within a tag". If the determination results in "YES", the process goes to S1511. If the determination results in "NO", the process goes to S1512.

In S1511, contents based on the explanatory information to a patient, which are displayed in the findings field 71c, the diagnosis field 71d, and the procedures field 71e of the observation tag 73 (73a, 73b, 73c, or 73d), which is clicked in the above described S1401, are changed to a simpler expression, and displayed. Namely, each underlined word within the contents is changed to a word of a document level, which becomes an expression of the next simpler level of the document level corresponded to the word, and displayed. Then, the process goes back to (4), namely, S1401.

In S1512, it is determined whether or not the item selected in S1509 is "more technical display of display field within a tag". If the determination results in "YES", the process goes to S1513. If the determination results in "NO", the process goes to S1514.

In S1513, contents based on the explanatory information to a patient, which are displayed in the findings field 71c, the diagnosis field 71d, and the procedures field 71e of the observation tag 73 (73a, 73b, 73c, or 73d), which is clicked in the above described S1401, are changed to a more technical expression, and displayed. Namely, each underlined word within the contents is changed to a word of a document level, which becomes an expression of the next more technical level of the document level corresponded to the word, and displayed. Then, the process goes back to (4), namely, S1401.

In S1514, it is proved that the item selected in S1509 is any of the document levels [1], [2], [3], [4], [5], and [6] of the "stage...". Therefore, the contents based on the explanatory information to a patient, which are displayed in the findings field 71c, the diagnosis field 71d, and the procedures field 71e of the observation tag 73 (73a, 73b, 73c, or 73d), which is clicked in the above described S1401, are changed to an expression according to the document level selected in the "stage...", and displayed. Namely, each underlined word within the contents is changed to a word according to the document level selected in the "stage...", and displayed. Then, the process goes back to (4), namely, S1401.

With such a process shown in Fig. 15B, contents based on the explanatory information to a patient, which are displayed in the findings field 71c, the diagnosis field 71d, and the procedures field 71e of a clicked observation tag (73a, 73b, 73c, or 73d) are changed to contents of a simpler or more technical expression, or to contents of an expression according to a desired document level, and displayed according to an item selected from the pop-up menu 84.

With the above described processes shown in Figs. 14A, 14B, 15A, and 15B, contents based on the explanatory information to a patient, which are displayed in the findings field 71c, the diagnosis field 71d, and the procedures field 71e for each of the observation tags 73 (73a, 73b, 73c, and 73d), can be changed to contents according to the medical knowledge level of a patient.

On the explanatory screen to a patient 70 shown in Fig. 13, for example, a button, with which an instruction can be made to allow a change in the contents displayed in the findings field 71c, the diagnosis field 71d, and the procedures field 71e of all of the observation tags 73 (73a, 73b, 73c, and 73d) at one time, may be provided, and the contents may be changed at one time with the press (click) of this button. In this case, pressing this button displays a pop-up menu with which any of items such as a simpler display, a more technical display, and a display made by changing to contents according to a selected document level is allowed to be selected, and a desired item is selected from the pop-up menu, so that the contents are changed to corresponding contents and displayed, similar to the pop-up menus shown in Figs. 17A and 17B.

Additionally, on the explanatory screen to a patient 70 shown in Fig. 13, an accompanying information screen 90 based on corresponding accompanying information is displayed with the press (click) of the accompanying information button 75. The accompanying information is information about a symptom, and preregistered (prerecorded) to the database of the server 15. Corresponding accompanying information is read out depending on need, and an accompanying information screen 90 based on the accompanying information is displayed.

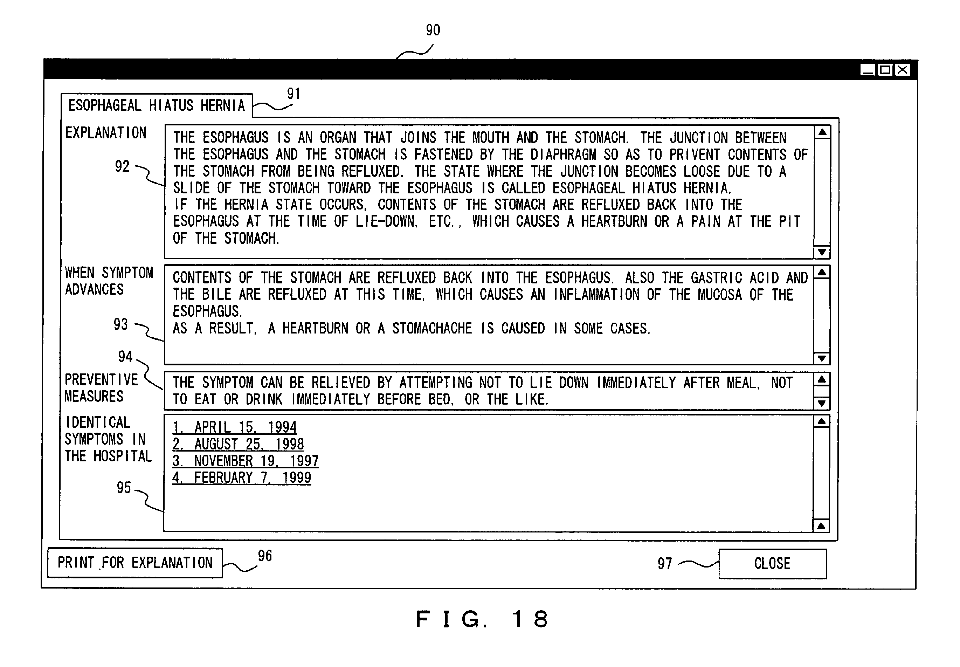

Fig. 18 exemplifies the accompanying information screen 90 displayed at this time.

As shown in this figure, on the accompanying information screen 90, a symptom title tag 91, an explanation field 92, an advanced symptom case field 93, a preventive measures field 94, an in-facility identical symptom case field 95, etc. are displayed.

The symptom title tag 91 is a tag that indicates a symptom that is significant in an examination. In this figure, only one symptom title tag 91 that indicates a symptom "esophageal hiatus hernia" is shown. Actually, however, tags the number of which is equal to the number of symptoms are created, and corresponding symptom title tags 91 are respectively displayed. Additionally, in this case, for each of the symptom title tags 91, according to a symptom, an explanation about the symptom, an explanation about the case where the symptom advances, an explanation about preventive measures against the symptom, an explanation about symptom cases identical to the symptom in the facility are respectively displayed in an explanation field 92, an advanced symptom case field 93, a preventive measures field 94, and an in-facility identical symptom case field 95. Accordingly, with the selection (click) of a desired symptom title tag 91, accompany information according to the symptom is displayed.

Additionally, in the in-facility identical symptom case field 95, a list of identical symptoms is displayed in descending order of severity if the identical symptoms exist in the facility. Each of the symptoms in the list is represented by year, month and day (a date) on which an examination for the identical symptom is conducted, and underlined. Besides, if a plurality of examinations are conducted for an identical symptom for one patient on different dates, the respective dates of the examinations are displayed in the in-facility identical symptom case field 95. A desired date is selected (clicked) from the list thus displayed, so that contents based on the information about the examination conducted on that date are displayed, and can be viewed. Only the dates are displayed in the in-facility identical case field 95 in this figure. However, also findings, diagnosis, etc., which are conducted in an examination, may be displayed along with a date.

Furthermore, with the press (click) of a print-for-explanation button 96, contents displayed on the accompanying information screen 90 are printed. Or, with the press (click) of a close button 97, the accompanying information screen 90 is closed.

Still further, with the press (click) of the print-for-explanation button 74 on the explanatory screen to a patient 70 shown in Fig. 13, or with the press (click) of the print-for-explanation button 41 on the input screen 30 shown in Fig. 4, etc., contents based on explanatory information to a patient are printed by the printer 19b (or 19, etc.).

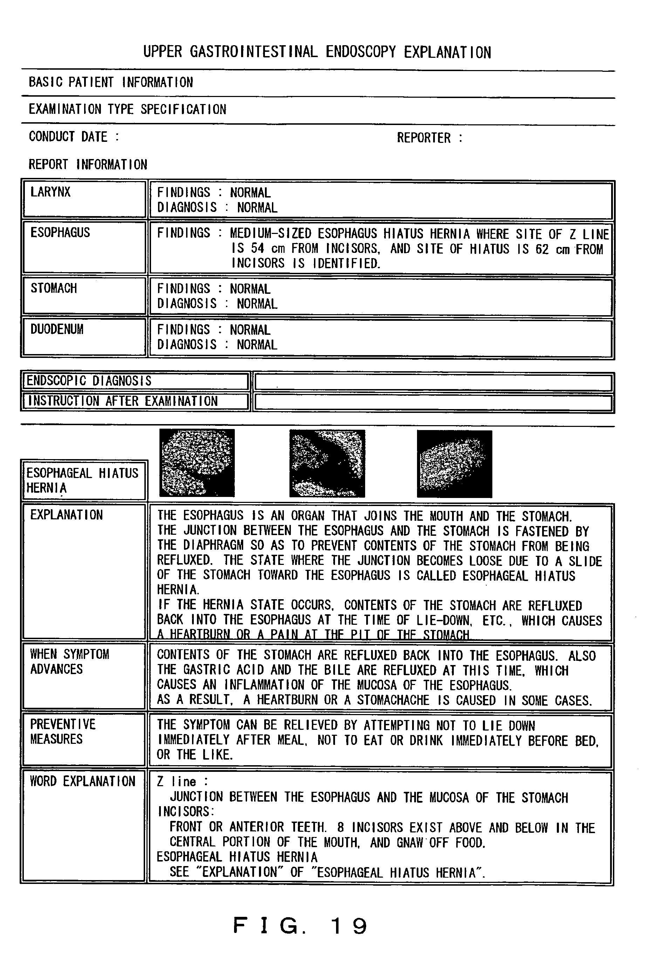

Fig. 19 exemplifies a printing result based on explanatory information to a patient, which is printed at this time.

As shown in this figure, printed as the printing result are: the basic patient information and the examination type specification, which are displayed in the display areas 31 and 32 on the input screen 30 shown in Fig. 4, etc.; contents displayed in the endoscopic diagnosis field 71a, the instruction after examination field 71b, the findings field 71c, the diagnosis field 71d, and the procedures field 71e of each of the observation tags 73 (73a, 73b, 73c, and 73d), and the endoscopic image field 72 on the explanatory screen to a patient 70 shown in Fig. 13; and contents in the explanation field 92, the advanced symptom case field 93, the preventive measures field 94, and the in-facility identical symptom case field 95 of each symptom title tag 91 on the accompanying information screen 90 shown in Fig. 18. Also the contents, etc. displayed in the word explanation window 81 shown in Fig. 16 are printed.

Settings of these printing items can be changed depending on need, although this is not shown.

Additionally, with the press (click) of the close button 76 on the explanatory screen to a patient 70 shown in Fig. 13, the explanatory screen to a patient 70 is closed, and at the same time, explanatory information to a patient according to the contents of the explanatory screen to a patient 70 at this time (at the time of pressing the close button 76) is stored in the database of the server 15. Note that this explanatory information to a patient is stored by being corresponded to created report information as described above. Besides, an average value of document levels is obtained based on the document levels of respective words used in the contents on the explanatory screen to a patient 70 at the time of pressing the close button 76, and this average value is stored in the database of the server 15 as information about the medical knowledge level of a patient. When explanatory information to a patient for the same patient is created the next time, it is created based on the information about the medical knowledge level of this patient. The information about the medical knowledge level of a patient is stored by being associated with the created report information. Accordingly, information about the medical knowledge level of a patient is stored each time an examination is conducted for the patient. Thereafter, the transition of the medical knowledge level of the patient can be confirmed, which can be helpful to a doctor's evaluation of whether or not an explanation is sufficiently given to the patient.

Additionally, when the explanatory screen to a patient 70 is displayed (activated) or closed (deactivated), an authentication process may be executed with an ID card possessed by a patient, or with the fingerprints of a patient. As a result, for example, a time required to give an explanation to a patient can be measured, which can be helpful to a doctor's evaluation of whether or not an explanation is sufficiently given. However, it may be configured so that the explanatory screen to a patient 70 can be forcibly activated or deactivated without an ID card in consideration of an emergency, etc. In this case, information indicating who activates and deactivates the screen is stored in the server 15 in order to identify a person who activates and deactivates the screen.

Additionally, a questionnaire on an explanation given by a doctor is conducted for a patient via the explanatory screen to a patient 70, and a questionnaire result can be used as one means for collecting a doctor's evaluation, which can help run a hospital. Such a questionnaire may be conducted in a way such that a patient answers such a questionnaire by logging in to an information terminal for patients, which is connected to the hospital-wide network 3, for example, while he or she is waiting to make a payment.

As described above, according to this preferred embodiment, explanatory information to a patient is also created at the same time that report information on an endoscopy is created, whereby hassle of separately creating material for explanation to a patient can be reduced, leading to an increase in the operation efficiency of a doctor. Additionally, since the explanatory information to a patient is created according to the medical knowledge level of a patient, it becomes possible to make the patient sufficiently understand contents of an examination, and to improve the understanding of the medical knowledge of the patient. Besides, in consequence, patients' reliability of a hospital is enhanced, leading to an increase in an effect of pulling patients owing to their revisits to a hospital.

This preferred embodiment is described by taking an endoscopy as an example. However, this preferred embodiment may be applied to examinations other than an endoscopy, such as a pathologic examination, an ultrasonic examination, a CT examination, an MRI examination, etc.

As described above, the medical information system and the medical information management method according to the present invention are described in detail. However, the present invention is not limited to the above described preferred embodiment. Various types of improvements and modifications may be made within the scope that does not deviate from the essence of the present invention, as a matter of course.

As described above in detail, according to the present invention, an increase in the operation efficiency of a doctor, improvements in the understanding of medical knowledge or medical care knowledge of a patient, and an increase in the effect of pulling patients owing to their revisits to a hospital can be implemented.

## Claims

1. A medical information system, comprising:
an inputting unit (22) inputting report information on an examination;
a first storing unit (15) storing the report information input by the inputting unit (22); and
a creating unit (26) creating explanatory information to a patient by using the report information.

2. The medical information system according to claim 1, further comprising:
a second storing unit (15) storing the explanatory information to a patient;
a displaying unit (24) displaying contents based on the explanatory information to a patient;
an instructing unit (22) instructing a character, a word, or a text within the contents based on the explanatory information to a patient, which is displayed by the displaying unit; and
a changing unit (26) changing the character, the word, or the text, which is instructed by the instructing unit, to a simpler or more technical character, word, or text.

3. The medical information system according to claim 2, further comprising:
a third storing unit (15) storing a document level based on a change made by the changing unit, and contents of the change made by the changing unit.

4. The medical information system according to claim 1, wherein
the creating unit (26) creates the explanatory information to a patient by using the report information input by the inputting unit (22).

5. The medical information system according to claim 3, wherein
the creating unit (26) creates the explanatory information to a patient by using the report information input by the inputting unit (22).

6. The medical information system according to claim 1, wherein
the creating unit (26) creates the explanatory information to a patient by using the report information stored in the first storing unit (15).

7. The medical information system according to claim 3, wherein
the creating unit (26) creates the explanatory information to a patient by using the report information stored in the first storing unit (15).

8. The medical information system according to claim 1, wherein
the inputting unit (22) inputs the information on the examination with a selection of a term, which is a sample text.

9. The medical information system according to claim 3, wherein
the inputting unit (22) inputs the information on the examination with a selection of a term, which is a sample text.

10. The medical information system according to claim 3, wherein
the creating unit (26) creates explanatory information to a patient by using the report information based on the document level.

11. The medical information system according to claim 3, wherein
a start and an end of an explanation to a patient is checked by starting and ending a display of contents based on the explanatory information to a patient with the use of an ID card individually given to a patient, and measuring a time required to give the explanation to the patient.

12. A medical information management method, comprising:
inputting report information on an examination;
storing the input report information; and
creating explanatory information to a patient by using the report information.

13. The medical information management method according to claim 12, further comprising:
storing the created explanatory information to a patient;
displaying contents based on the explanatory information to a patient;
instructing a character, a word, or a text within the displayed contents based on the explanatory information to a patient; and
changing the instructed character, word, or text to a simpler or more technical character, word, or text.

14. The medical information management method according to claim 13, further comprising
storing a document level based on a change to a simpler or more technical character, word, or text, and contents of the change.
